# EUROPEAN PATENT SPECIFICATION

(11) **EP 4 076 358 B1**
(45) Date of publication and mention of the grant of the patent: **18.06.2025**
(21) Application number: 20821227.4
(22) Date of filing: 14.12.2020
(51) Int. Cl.: A61K 8/31, A61K 8/34, A61K 8/39, A61K 8/41, A61K 8/73, A61K 8/9789, A61Q 5/02, A61Q 5/12

(54) **AQUEOUS COMPOSITION FOR TREATING KERATIN FIBRES, COMPRISING A PLANT OIL, A HYDROCARBON-BASED OIL, A GLYCEROLATED NONIONIC SURFACTANT, A POLYSACCHARIDE AND A SOLVENT**
WÄSSRIGE ZUSAMMENSETZUNG ZUR BEHANDLUNG VON KERATINFASERN MIT EINEM PFLANZENÖL, EINEM ÖL AUF KOHLENWASSERSTOFFBASIS, EINEM GLYCEROLIERTEN NICHTIONISCHEN TENSID, EINEM POLYSACCHARID UND EINEM LÖSUNGSMITTEL
COMPOSITION AQUEUSE POUR LE TRAITEMENT DE FIBRES DE KÉRATINE, COMPRENANT UNE HUILE VÉGÉTALE, UNE HUILE HYDROCARBONÉE, UN TENSIOACTIF NON IONIQUE GLYCÉROLÉ, UN POLYSACCHARIDE ET UN SOLVANT

(30) Priority: 17.12.2019 FR 1914580
(43) Date of publication of application: 26.10.2022
(73) Proprietor: L'OREAL, 75008 Paris (FR)
(72) Inventor: LE CHAUX, Virginie, 93400 SAINT OUEN (FR); MALOUG, Saber, 93400 SAINT OUEN (FR)
(74) Representative: Casalonga
(86) International application number: PCT/EP2020/085901
(87) International publication number: WO 2021/122412

(56) References cited:
- FR-A1- 2 902 320
- FR-A1- 2 965 482
- DATABASE GNPD [online] MINTEL; 26 September 2019 (2019-09-26), ANONYMOUS: "Moisturizing Conditioner with Quinoa", XP055724815, retrieved from www.gnpd.com Database accession no. 6902347
- DATABASE GNPD [online] MINTEL; 15 July 2015 (2015-07-15), ANONYMOUS: "After Sun Nourishing Hair Treatment", XP055724913, retrieved from www.gnpd.com Database accession no. 3295129
- DATABASE GNPD [online] MINTEL; 21 November 2019 (2019-11-21), ANONYMOUS: "Shine Shampoo", XP055724876, retrieved from www.gnpd.com Database accession no. 6637539

## Description

The present invention relates to a composition comprising one or more plant oils, one or more hydrocarbon-based oils other than plant oils, one or more glycerolated nonionic surfactants, one or more polysaccharides, one or more C₁ to C₆ monoalcohols and water.

The invention also relates to a process for treating keratin fibres, in particular human keratin fibres such as the hair, comprising at least one step of applying a composition according to the invention to said keratin fibres.

It is well known that hair may be sensitized or embrittled to varying degrees as a result of the action of atmospheric agents such as light, water and moisture, and also repeated mechanical or chemical treatments such as brushing, combing, washing, bleaching, permanent waving, relaxing and/or dyeing. These attacking factors impair the hair fibre and reduce its mechanical properties such as the tensile strength, the breaking load and the elasticity, or its resistance to swelling in an aqueous medium. The hair is dull, coarse and brittle. The hair is difficult to disentangle and to style.

Substances for protecting the hair against such degradation have been sought for many years in the cosmetics industry; products that improve the cosmetic properties, notably the disentangling, soft feel and volume of the head of hair, and that preserve or reinforce the intrinsic mechanical properties of keratin fibres, such as the tensile strength, the breaking load and the elasticity, or their resistance to swelling in an aqueous medium, are sought in particular.

Thus, care/conditioning compositions have been proposed, notably for sensitized hair, which comprise one or more silicones, to obtain acceptable cosmetic performance qualities.

However, these compositions have several drawbacks: presence of silicone, the environmental profile (biodegradability, water footprint) of which is not always optimal, generally opaque (sparingly aesthetic) appearance of the composition associated with the presence of silicone, and rapid regreasing of the hair accompanied by lankness.

In addition, repeated applications of these compositions often have the effect of giving the hair an unpleasant feel, loss of volume and liveliness of the head of hair, and occasionally lack of sheen.

These observations gave rise to the interest in developing a care/conditioning composition which can, where appropriate, be formulated without silicone (silicone-free), while at the same time having improved working qualities and good cosmetic properties, and which is capable of repairing/conditioning keratin fibres without making them lank, so as to give them good conditioning properties. This composition must in particular make it possible to treat sensitized, embrittled or damaged keratin fibres, and more particularly fine hair.

These objectives are achieved by the present invention, one subject of which is notably a composition comprising:
(i) one or more plant oils,
(ii) one or more hydrocarbon-based oils other than the plant oils (i),
(iii) one or more glycerolated nonionic surfactants,
(iv) one or more polysaccharides,
(v) one or more C₁ to C₆ monoalcohols and
(vi) water; and
the sum of the total content of plant oil(s) (i) and of the total content of hydrocarbon-based oil(s) (ii) being greater than or equal to 5% by weight, relative to the total weight of the composition.

A silicone free commercial moisturizing conditioner comprising (i) Simmondsia Chinensis seed oil, prunus amygdalus dulcis oil and glycine soja oil, (ii) squalene, (iii) polygycleryl-3 diisostearate, (iv) xanthan gum and guar hydroxypropyltrimonium chloride, (v) isopropanol and (vi) water, is known from the prior art (Database GNPD Mintel, Record ID 6902347). But the content of the plant oils (i) and of the squalene (ii) is not described.

It has been found that hair treated with the composition according to the invention is particularly clean and has good cosmetic properties. Hair thus treated is particularly light, soft- and smooth-feeling, shiny, easy to disentangle, more manageable, and has good volume and also repaired ends.

Furthermore, it has been observed that the composition according to the invention gives the hair (mechanical) strength.

Moreover, the Applicant has also found that the use of the composition according to the invention makes it possible to substantially reduce its environmental impact relative to a conventional washing and conditioning composition.

A subject of the invention is also a treatment process, preferably for caring for/conditioning keratin fibres, in particular human keratin fibres such as the hair, comprising at least one step of applying to said keratin fibres a composition according to the invention.

The invention also relates to the use of the composition according to the invention for treating keratin fibres, and more particularly for caring for/conditioning keratin fibres.

Other characteristics, aspects and advantages of the invention will emerge even more clearly on reading the description and the example that follows.

In the present description, and unless otherwise indicated:
- the term "at least one" is equivalent to the expression "one or more" and can be replaced therewith;
- the term "between" is equivalent to the expression "ranging from" and can be replaced therewith, and implies that the limits are included;
- the term "keratin fibres", according to the present patent application, preferably denotes human keratin fibres and more particularly the hair.

Preferably, the composition according to the invention is silicone-free.

The term "silicone-free" means that the composition according to the invention does not comprise any silicone, or that the silicone(s) that may be present in the composition according to the invention are included in a total content of less than or equal to 0.1% by weight, preferably less than 0.05% by weight relative to the total weight of the composition according to the invention, and better still is free of silicone (0% by weight).

The term "silicone" means any organosilicon polymer or oligomer of linear or cyclic and branched or crosslinked structure, of variable molecular weight, obtained by polymerization and/or polycondensation of suitably functionalized silanes and essentially constituted of a repetition of main units in which the silicon atoms are connected to each other via oxygen atoms (siloxane bond -Si-O-Si-), optionally substituted hydrocarbon-based radicals being connected directly to said silicon atoms via a carbon atom; and more particularly dialkylsiloxane polymers, amino silicones and dimethiconols.

### The plant oils (i)

The composition according to the present invention comprises one or more plant oils.

The term "oil" means any fatty substance that is in liquid form at room temperature (25°C) and at atmospheric pressure (1.013 × 10⁵ Pa).

The term "fatty substance" means an organic compound that is insoluble in water at room temperature (25°C) and at atmospheric pressure (1.013×10⁵ Pa) (solubility of less than 5% by weight, preferably less than 1% by weight and even more preferentially less than 0.1% by weight). They have in their structure at least one hydrocarbon-based chain including at least 6 carbon atoms. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

Preferably, the plant oil(s) present in the composition according to the invention are chosen from oils of plant origin such as phytostearyl esters, such as phytostearyl oleate, phytostearyl isostearate and lauroyl/octyldodecyl/phytostearyl glutamate, for example sold under the name Eldew PS203 by Ajinomoto, triglycerides constituted of fatty acid esters of glycerol, the fatty acids of which may have chain lengths ranging from C₄ to C₂₄, these chains possibly being linear or branched, and saturated or unsaturated; these oils are notably heptanoic or octanoic triglycerides, sweet almond oil, argan oil, avocado oil, groundnut oil, camellia oil, safflower oil, beauty-leaf oil, rapeseed oil, coconut oil (or coconut kernel oil), coriander oil, marrow oil, wheatgerm oil, jojoba oil, linseed oil, macadamia oil, corn germ oil, hazelnut oil, walnut oil, vernonia oil, apricot kernel oil, olive oil, evening primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil; or alternatively caprylic/capric acid triglycerides, for instance those sold by the company Stéarinerie Dubois or those sold under the names Miglyol 810^{®}, 812^{®} and 818^{®} by the company Dynamit Nobel, and the refined plant-based perhydrosqualene sold under the name Fitoderm by the company Cognis; the plant-based squalene sold, for example, under the name Squalive by the company Biosynthis.

The plant oils that may be used according to the invention are neither silicone-based nor oxyalkylenated (i.e. in particular neither oxypropylenated nor oxyethylenated).

According to a preferred embodiment of the invention, the plant oil(s) are chosen from sweet almond oil, argan oil, avocado oil, groundnut oil, camellina oil, safflower oil, beauty-leaf oil, rapeseed oil, coconut oil (or coconut kernel oil), coriander oil, marrow oil, wheat germ oil, jojoba oil, linseed oil, macadamia oil, corn germ oil, hazelnut oil, walnut oil, vernonia oil, apricot kernel oil, olive oil, evening-primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil, and mixtures thereof; more preferentially from soybean oil, jojoba oil, castor oil and coconut oil (or coconut kernel oil), and mixtures thereof.

In a particular embodiment of the invention, the composition comprises jojoba oil.

Preferably, the total content of plant oil(s) present in the composition according to the invention is between 0.1% and 20% by weight, more preferentially between 1% and 15% by weight, even more preferentially between 3% and 10% by weight, relative to the total weight of the composition.

### The hydrocarbon-based oils (ii)

The composition according to the present invention comprises one or more hydrocarbon-based oils other than the plant oils (i).

For the purposes of the invention, the term "hydrocarbon-based oil" means an oil mainly containing hydrogen and carbon atoms and possibly one or more oxygen, nitrogen, sulfur and/or phosphorus atoms. In particular, a hydrocarbon-based oil according to the invention may optionally include one or more functions chosen from hydroxyl, ester, ether and carboxylic functions.

For the purposes of the invention, a hydrocarbon-based oil does not comprise any silicon atoms.

Generally, the hydrocarbon-based oil (ii) according to the invention has a viscosity of from 0.5 to 100 000 mPa.s, preferably from 50 to 50 000 mPa.s and more preferably from 100 to 30 000 mPa.s, measured at a temperature of 25°C using a Rheomat^{®} RM 180 viscometer at a shear rate of 1 s⁻¹.

The hydrocarbon-based oils (ii) according to the invention are not of plant origin.

Furthermore, the hydrocarbon-based oils (ii) according to the invention are different from the plant oils (i) described above, and from the glycerolated nonionic surfactants (iii), from the C₁ to C₇ polyols, from the non-oxyalkylenated and non-glycerolated C₈ to C₃₀ fatty alcohols and from the C₁ to C₆ monoalcohols described below.

Preferably, the hydrocarbon-based oil(s) (ii) have a surface tension of less than or equal to 50 mN/m, more preferentially less than or equal to 30 mN/m.

The surface tension is measured at room temperature (25°C) and at atmospheric pressure (1.013×10⁵ Pa).

In a manner known per se, the surface tension may be measured using a Wilhelmy plate tensiometer (standard NF EN 14370).

For information purposes, the surface tension values of certain hydrocarbon-based oils are given below, measured at room temperature (25°C) and at atmospheric pressure (1.013×10⁵ Pa):
2,2,4,6,6-pentamethylpentane (21.6 mN/m), 2,2,4,4,6,8,8-heptamethylnonane (24.2 mN/m), dodecane (24.8 mN/m), hemisqualane (24.9 mN/m), isodecyl neopentanoate (25.4 mN/m), cocoyl caprylate (28.3 mN/m), dioctyl ether (27.1 mN/m), isopropyl myristate (28.3 mN/m), tetradecane (26 mN/m).

Preferably, the hydrocarbon-based oil(s) (ii) have an evaporation rate of greater than 0.5% after 30 minutes.

The evaporation rate of a hydrocarbon-based oil in accordance with the invention may notably be evaluated by means of the protocol described in WO 06/013 413, and more particularly by means of the protocol described below.
15 g of hydrocarbon-based oil are placed in a crystallizing dish (diameter: 7 cm) placed on a balance that is in a chamber of about 0.3 m³ with regulated temperature (25°C) and hygrometry (50% relative humidity).

The hydrocarbon-based oil is allowed to evaporate freely, without stirring, while providing ventilation by means of a fan (Papst-Motoren, reference 8550 N, rotating at 2700 rpm) placed vertically above the crystallizing dish containing the hydrocarbon-based oil, the blades being directed toward the crystallizing dish, 20 cm away from the bottom of the crystallizing dish.

The mass of hydrocarbon-based oil remaining in the crystallizing dish is measured at regular intervals.

The evaporation profile of the hydrocarbon-based oil is then obtained by plotting the curve of the amount of product evaporated (in mg/cm²) as a function of time (in minutes).

The evaporation rate is then calculated, which corresponds to the tangent to the origin of the curve obtained. The evaporation rates are expressed in mg of hydrocarbon-based oil evaporated per unit area (cm²) and per unit time (minutes).

The hydrocarbon-based oils are advantageously chosen from:
(a) alkanes containing from 8 to 16 carbon atoms, and notably:
   - branched C₈ to C₁₆ alkanes, for instance C₈ to C₁₆ isoalkanes of petroleum origin (also known as isoparaffins), for instance isododecane (also known as 2,2,4,4,6-pentamethylheptane), isodecane, isohexadecane and, for example, the oils sold under the trade name Isopar or Permethyl,
   - linear C₈ to C₁₆ alkanes, for instance n-dodecane (C₁₂) and n-tetradecane (C₁₄) sold by Sasol under the references, respectively, Parafol 12-97 and Parafol 14-97, and also mixtures thereof, the undecane-tridecane mixture, mixtures of n-undecane (C₁₁) and of n-tridecane (C₁₃) obtained in examples 1 and 2 of patent application WO 2008/155059 from the company Cognis, and mixtures thereof;
(b) short-chain esters (containing from 3 to 8 carbon atoms in total) such as ethyl acetate, methyl acetate, propyl acetate or n-butyl acetate;
(c) synthetic ethers containing from 10 to 40 carbon atoms, such as dicaprylyl ether;
(d) linear or branched hydrocarbons of mineral or synthetic origin comprising more than 16 carbon atoms, such as petroleum jelly, polydecenes, hydrogenated polyisobutene such as Parleam^{®}, squalane, perhydrosqualene and liquid paraffins, and mixtures thereof;
(e) synthetic esters such as oils of formula R₁COOR₂ in which R₁ represents a linear or branched fatty acid residue including from 1 to 40 carbon atoms and R₂ represents a, notably branched, hydrocarbon-based chain containing from 1 to 40 carbon atoms, on condition that R₁ + R₂ ≥ 10, for instance purcellin oil (cetostearyl octanoate), butyl stearate, 2-diethylhexyl succinate, isopropyl myristate, isopropyl palmitate, C₁₂ to C₁₅ alkyl benzoates, hexyl laurate, diisopropyl adipate, isononyl isononanoate, 2-ethylhexyl palmitate, isostearyl isostearate, 2-hexyldecyl laurate, 2-octyldecyl palmitate, 2-octyldodecyl myristate, alcohol or polyalcohol heptanoates, octanoates, decanoates or ricinoleates such as propylene glycol dioctanoate; hydroxylated esters such as isostearyl lactate, diisostearyl malate and 2-octyldodecyl lactate; polyol esters and pentaerythritol esters; triglyceride esters such as caprylic/capric triglyceride.

Preferably, the composition according to the invention comprises one or more C₈ to C₁₆ hydrocarbon-based oils; more preferentially, the hydrocarbon-based oil(s) are chosen from branched C₈ to C₁₆ alkanes such as isododecane, isodecane and isohexadecane, linear C₈ to C₁₆ alkanes such as n-dodecane and n-tetradecane, and mixtures thereof.

Preferably, the total content of hydrocarbon-based oil(s) present in the composition according to the invention is between 0.1% and 40% by weight, more preferentially between 1% and 30% by weight, even more preferentially between 5% and 25% by weight and better still between 10% and 20% by weight, relative to the total weight of the composition.

More preferentially, the total content of C₈ to C₁₆ hydrocarbon-based oil(s) present in the composition according to the invention is between 0.1% and 40% by weight, more preferentially between 1% and 30% by weight, even more preferentially between 5% and 25% by weight or even between 10% and 20% by weight, relative to the total weight of the composition.

The sum of the total content of plant oil(s) (i) and of the total content of hydrocarbon-based oil(s) (ii) is greater than or equal to 5% by weight, relative to the total weight of the composition.

Preferably, the sum of the total content of plant oil(s) (i) and of the total content of hydrocarbon-based oil(s) (ii) is between 10% and 40% by weight, more preferentially between 15% and 35% by weight, better still between 15% and 25% by weight, relative to the total weight of the composition.

More preferentially, the sum of the total content of plant oil(s) (i) and of the total content of C₈ to C₁₆ hydrocarbon-based oil(s) (ii) is greater than or equal to 5% by weight; even more preferentially, said sum is between 10% and 40% by weight, better still between 15% and 35% by weight, or even between 15% and 25% by weight, relative to the total weight of the composition.

### The glycerolated nonionic surfactants (iii)

The composition according to the invention comprises one or more glycerolated nonionic surfactants.

For the purposes of the invention, the term *"glycerolated nonionic surfactant"* means a nonionic surfactant comprising at least one mole of glycerol, preferably comprising a number of moles of glycerol ranging from 1 to 50, more preferentially from 1 to 20 and even more preferentially from 2 to 10.

Examples of glycerolated nonionic surfactants that are preferably used, alone or as a mixture, include:
- monoesters or polyesters of linear or branched, monoglycerolated or polyglycerolated C₈ to C₄₀ acids, comprising from 1 to 50 mol of glycerol, preferably from 1 to 20 or even from 2 to 10 mol of glycerol; in particular monoesters or diesters of linear or branched C₈ to C₃₂, better still C₁₀ to C₂₈ or even C₁₀ to C₂₄ acids, comprising from 1 to 50 mol of glycerol, preferably from 1 to 20 or even from 2 to 10 mol of glycerol;
- linear or branched, saturated or unsaturated, monoglycerolated or polyglycerolated C₈ to C₄₀, better still C₁₀ to C₂₈, even better still C₁₀ to C₂₄ or even C₁₀ to C₁₈ alcohols, preferably including one or two fatty chains, and comprising from 1 to 50 mol of glycerol, preferably from 1 to 20 or even from 2 to 10 mol of glycerol.

Preferably, the glycerolated nonionic surfactant(s) according to the invention do not comprise any oxyalkylenes units, such as oxyethylenes or oxypropylenes.

Monoesters or diesters of linear or branched, monoglycerolated or polyglycerolated C₈ to C₄₀, more preferentially C₈ to C₃₂, better still C₁₀ to C₂₈ or even C₁₀ to C₂₄ acids, comprising from 1 to 50 mol of glycerol, preferably from 1 to 20 or even from 2 to 10 mol of glycerol, will be preferred most particularly, alone or as a mixture; and better still, alone or as a mixture:
- diesters of branched C₁₂ to C₃₂, better still C₁₄ to C₂₈, or even C₁₆ to C₂₄ acids, comprising from 1 to 50 mol of glycerol, preferably from 1 to 20 or even from 2 to 10 mol of glycerol; preferentially diesters of branched C₁₆ to C₂₄ acids, comprising from 2 to 10 mol of glycerol, among which mention may be made of isostearic acid diester containing 3 mol of glycerol (INCI name: Polyglyceryl-3 diisostearate);
- monoesters of linear or branched, preferably linear, C₈ to C₂₄, better still C₁₀ to C₂₀, or even C₁₀ to C₁₈ acids, comprising from 1 to 50 mol of glycerol, preferably from 1 to 20 or even from 2 to 10 mol of glycerol; preferentially monoesters of linear C₁₀ to C₁₈ acids, comprising from 2 to 10 mol of glycerol, among which mention may be made of lauric acid monoester containing 4 mol of glycerol (INCI name: Polyglyceryl-4 laurate).

Preferably, the glycerolated nonionic surfactant(s) (iii) represent from 0.01% to 20% by weight, more preferentially from 0.1% to 10% by weight and even more preferentially from 0.3% to 5% by weight relative to the total weight of the composition.

Preferably, when one or more (poly)glycerol (poly)esters of C₈ to C₄₀ acids are present in the composition according to the invention, the monoglycerolated or polyglycerolated monoesters or polyesters of linear or branched C₈ to C₄₀ acids, comprising from 1 to 50 mol of glycerol, represent from 0.01% to 20% by weight, more preferentially from 0.1% to 10% by weight and even more preferentially from 0.3% to 5% by weight relative to the total weight of the composition.

According to a preferred embodiment of the invention, the weight ratio of the total content of plant oil(s) (i) as described previously, on the one hand, to the total content of glycerolated nonionic surfactant(s) (iii) as described previously, on the other hand, is greater than or equal to 1, more preferentially greater than or equal to 2 and even more preferentially between 2 and 4.

According to another preferred embodiment of the invention, the weight ratio of the total content of plant oil(s) (i) as described previously, on the one hand, to the total content of monoglycerolated or polyglycerolated monoesters or polyesters of linear or branched C₈ to C₄₀ acids, comprising from 1 to 50 mol of glycerol as described previously, on the other hand, is greater than or equal to 1, more preferentially greater than or equal to 2 and even more preferentially between 2 and 4.

According to a particular embodiment of the invention, the composition according to the invention may also comprise one or more additional nonionic surfactants other than the glycerolated nonionic surfactants (iii) as described above.

Examples of additional nonionic surfactants that may be mentioned include the following nonionic surfactants:
- oxyalkylenated (C₈-C₂₄)alkylphenols;
- saturated or unsaturated, linear or branched C₈ to C₄₀ alcohols, preferably including one or two fatty chains;
- saturated or unsaturated, linear or branched, oxyalkylenated C₈ to C₃₀ fatty acid amides;
- esters of saturated or unsaturated, linear or branched, C₈-C₃₀ acids and of polyethylene glycols;
- preferably oxyethylenated esters of saturated or unsaturated, linear or branched, C₈ to C₃₀ acids and of sorbitol;
- esters of fatty acids and of sucrose;
- saturated or unsaturated oxyethylenated plant oils;
- condensates of ethylene oxide and/or of propylene oxide;
- N-(C₈-C₃₀)alkylglucamine and N-(C₈-C₃₀)acylmethylglucamine derivatives;
- aldobionamides;
- amine oxides;
- and mixtures thereof.

The oxyalkylene units are more particularly oxyethylene or oxypropylene units, or a combination thereof, preferably oxyethylene units.

The number of moles of ethylene oxide and/or of propylene oxide preferably ranges from 1 to 250, more particularly from 2 to 100 and better still from 2 to 50.

Preferably, the composition according to the invention is free of additional nonionic surfactants (other than the glycerolated nonionic surfactants (iii)) such as those described above.

Preferably, the total content of nonionic surfactant(s) ranges from 0.01% to 20% by weight, more preferentially from 0.1% to 10% by weight and even more preferentially from 0.3% to 5% by weight, relative to the total weight of the composition.

### The polysaccharides

The composition according to the invention comprises one or more polysaccharides.

According to the invention, the polysaccharides are also referred to as polymers bearing sugar units.

For the purposes of the present invention, the term "sugar unit" means an oxygen-bearing hydrocarbon-based compound containing several alcohol functions, with or without aldehyde or ketone functions, and which includes at least 4 carbon atoms.

The sugar units may be optionally modified by substitution, and/or by oxidation and/or by dehydration.

The sugar units of the thickening polymers are preferably derived from the following sugars: glucose, galactose, arabinose, rhamnose, mannose, xylose, fucose, anhydrogalactose, galacturonic acid, glucuronic acid, mannuronic acid, galactose sulfate, anhydrogalactose sulfate and fructose.

Preferably, the polysaccharide(s) that may be used in the composition according to the invention are chosen from non-associative polysaccharides, associative polysaccharides, and mixtures thereof.

Non-associative polysaccharides that may notably be mentioned include native gums such as:
a) tree or shrub exudates, including:
   - acacia gum (branched polymer of galactose, arabinose, rhamnose and glucuronic acid);
   - ghatti gum (polymer derived from arabinose, galactose, mannose, xylose and glucuronic acid);
   - karaya gum (polymer derived from galacturonic acid, galactose, rhamnose and glucuronic acid);
   - gum tragacanth (or tragacanth) (polymer of galacturonic acid, galactose, fucose, xylose and arabinose);
b) gums derived from algae, including:
   - agar (polymer derived from galactose and anhydrogalactose);
   - alginates (polymers of mannuronic acid and of glucuronic acid);
   - carrageenans and furcellerans (polymers of galactose sulfate and of anhydrogalactose sulfate);
c) gums derived from seeds or tubers, including:
   - guar gum (polymer of mannose and galactose);
   - locust bean gum (polymer of mannose and galactose);
   - fenugreek gum (polymer of mannose and galactose);
   - tamarind gum (polymer of galactose, xylose and glucose);
   - konjac gum (polymer of glucose and mannose);
d) microbial gums, including:
   - xanthan gum (polymer of glucose, mannose acetate, mannose/pyruvic acid and glucuronic acid);
   - gellan gum (polymer of partially acylated glucose, rhamnose and glucuronic acid);
   - scleroglucan gum (glucose polymer);
e) plant extracts, including:
   - cellulose (glucose polymer);
   - starch (glucose polymer) and
   - inulin.

These polysaccharides may be physically or chemically modified. As physical treatment, mention may notably be made of the temperature.

Chemical treatments that may be mentioned include esterification, etherification, amidation and oxidation reactions. These treatments make it possible to produce polymers that may notably be nonionic, anionic or amphoteric.

Preferably, these chemical or physical treatments are applied to guar gums, locust bean gums, starches and celluloses.

The nonionic guar gums that may be used according to the invention may be modified with C₁-C₆ (poly)hydroxyalkyl groups.

Among the C₁-C₆ (poly)hydroxyalkyl groups, mention may be made, by way of example, of hydroxymethyl, hydroxyethyl, hydroxypropyl and hydroxybutyl groups.

These guar gums are well known from the prior art and may be prepared, for example, by reacting corresponding alkene oxides, for instance propylene oxides, with the guar gum so as to obtain a guar gum modified with hydroxypropyl groups.

The degree of hydroxyalkylation preferably ranges from 0.4 to 1.2 and corresponds to the number of alkylene oxide molecules consumed by the number of free hydroxyl functional groups present on the guar gum.

Such nonionic guar gums optionally modified with hydroxyalkyl groups are sold, for example, under the trade names Jaguar HP8, Jaguar HP60 and Jaguar HP120 by the company Rhodia Chimie.

Use may also be made of cationic galactomannan gums such as those described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, a chloride). Such products are notably sold under the names Jaguar C13 S, Jaguar C 15, Jaguar C 17 and Jaguar C162 by the company Rhodia.

The botanical origin of the starch molecules that may be used in the present invention may be cereals or tubers. Thus, the starches are chosen, for example, from corn starch, rice starch, cassava starch, barley starch, potato starch, wheat starch, sorghum starch and pea starch.

The starches may be chemically or physically modified, notably by one or more of the following reactions: pregelatinization, oxidation, crosslinking, esterification, etherification, amidation, heat treatments.

Distarch phosphates or compounds rich in distarch phosphate will preferentially be used, for instance the product sold under the references Prejel VA-70-T AGGL (gelatinized hydroxypropyl cassava distarch phosphate), Prejel TK1 (gelatinized cassava distarch phosphate) and Prejel 200 (gelatinized acetylated cassava distarch phosphate) by the company Avebe, or Structure Zea from National Starch (gelatinized corn distarch phosphate).

According to the invention, amphoteric starches may also be used, these amphoteric starches comprising one or more anionic groups and one or more cationic groups. The anionic and cationic groups may be bonded to the same reactive site of the starch molecule or to different reactive sites; they are preferably bonded to the same reactive site. The anionic groups may be of carboxylic, phosphate or sulfate type, preferably carboxylic. The cationic groups may be of primary, secondary, tertiary or quaternary amine type.

The starch molecules may be derived from any plant source of starch, notably such as corn, potato, oat, rice, tapioca, sorghum, barley or wheat. It is also possible to use hydrolysates of the starches mentioned above. The starch is preferably derived from potato.

The non-associative polysaccharides of the invention may be cellulose-based polymers not including a C₁₀ to C₃₀ fatty chain in their structure.

According to the invention, the term *"cellulose-based* polymer" refers to any polysaccharide compound having in its structure sequences of glucose residues linked together via β-1,4 bonds; in addition to unsubstituted celluloses, the cellulose derivatives may be anionic, cationic, amphoteric or nonionic.

Thus, the cellulose-based polymers that may be used according to the invention may be chosen from unsubstituted celluloses, including those in a microcrystalline form, and cellulose ethers.

Among these cellulose-based polymers, cellulose ethers, cellulose esters and cellulose ester ethers are distinguished.

Among the cellulose esters are inorganic esters of cellulose (cellulose nitrates, sulfates, phosphates, etc.), organic esters of cellulose (cellulose monoacetates, triacetates, amidopropionates, acetatebutyrates, acetatepropionates and acetatetrimellitates, etc.), and mixed organic/inorganic esters of cellulose, such as cellulose acetatebutyrate sulfates and cellulose acetatepropionate sulfates. Among the cellulose ester ethers, mention may be made of hydroxypropylmethylcellulose phthalates and ethylcellulose sulfates.

Among the nonionic cellulose ethers without a C₁₀-C₃₀ fatty chain, i.e. which are *"non-associative",* mention may be made of (C₁-C₄)alkylcelluloses, such as methylcelluloses and ethylcelluloses (for example, Ethocel Standard 100 Premium from Dow Chemical); (poly)hydroxy(C₁-C₄)alkylcelluloses, such as hydroxymethylcelluloses, hydroxyethylcelluloses (for example, Natrosol 250 HHR sold by Aqualon) and hydroxypropylcelluloses (for example, Klucel EF from Aqualon); mixed (poly)hydroxy(C₁-C₄)alkyl-(C₁-C₄)alkylcelluloses, such as hydroxypropylmethylcelluloses (for example, Methocel E4M from Dow Chemical), hydroxyethylmethylcelluloses, hydroxyethylethylcelluloses (for example, Bermocoll E 481 FQ from Akzo Nobel) and hydroxybutylmethylcelluloses.

Among the anionic cellulose ethers without a fatty chain, mention may be made of (poly)carboxy(C₁-C₄)alkylcelluloses and salts thereof. Examples that may be mentioned include carboxymethylcelluloses, carboxymethylmethylcelluloses (for example Blanose 7M from the company Aqualon) and carboxymethylhydroxyethylcelluloses, and the sodium salts thereof.

Among the cationic cellulose ethers without a fatty chain, mention may be made of cationic cellulose derivatives such as cellulose copolymers or cellulose derivatives grafted with a water-soluble quaternary ammonium monomer, and notably described in patent US 4 131 576, such as (poly)hydroxy(C₁-C₄)alkylcelluloses, for instance hydroxymethyl-, hydroxyethyl- or hydroxypropylcelluloses notably grafted with a methacryloylethyltrimethylammonium, methacrylamidopropyltrimethylammonium or dimethyldiallylammonium salt. The commercial products corresponding to this definition are more particularly the products sold under the names Celquat^{®} L 200 and Celquat^{®} H 100 by the company National Starch.

Use may also be made of cellulose ether derivatives including quaternary ammonium groups such as the compounds notably described in FR 1 492 597; mention may be made of the polymers sold under the name Ucare Polymer JR (JR 400 LT, JR 125 and JR 30M) or LR (LR 400 and LR 30M) by the company Amerchol.

According to a preferred embodiment of the invention, the non-associative polysaccharide(s) are chosen from microbial gums; more preferentially from xanthan gum, scleroglucan gum, and mixtures thereof.

According to this embodiment, the total content of non-associative polysaccharide(s) present in the composition according to the invention is preferably between 0.01% and 10% by weight, more preferentially between 0.05% and 5% by weight and even more preferentially between 0.1% and 1% by weight, relative to the total weight of the composition.

Polysaccharides according to the invention that may also be mentioned include associative polysaccharides.

It is recalled that "associative polymers" are polymers that are capable, in an aqueous medium, of reversibly associating with each other or with other molecules.

Their chemical structure more particularly comprises at least one hydrophilic zone and at least one hydrophobic zone.

The term "hydrophobic group" means a radical or polymer with a saturated or unsaturated, linear or branched hydrocarbon-based chain, comprising at least 10 carbon atoms, preferably from 10 to 30 carbon atoms, in particular from 12 to 30 carbon atoms and more preferentially from 18 to 30 carbon atoms.

Preferentially, the hydrocarbon-based group is derived from a monofunctional compound. By way of example, the hydrophobic group may be derived from a fatty alcohol such as stearyl alcohol, dodecyl alcohol or decyl alcohol. It may also denote a hydrocarbon-based polymer, for instance polybutadiene.

The associative polysaccharides that may be used according to the invention may be chosen from nonionic associative polysaccharides, preferably from nonionic associative celluloses and nonionic associative galactomannan gums.

Preferentially, the nonionic associative polysaccharides are chosen from:
(1) celluloses modified with groups including at least one fatty chain, preferably from:
   - hydroxyethylcelluloses modified with groups including at least one fatty chain, such as alkyl, arylalkyl or alkylaryl groups, or mixtures thereof, and in which the alkyl groups are preferably C₈ to C₂₂, for instance the cetylhydroxyethylcellulose sold notably under the reference Natrosol Plus Grade 330 CS (C₁₆ alkyls) sold by the company Ashland, or the product Polysurf 67CS sold by the company Ashland,
   - hydroxyethylcelluloses modified with polyalkylene glycol ether alkyl phenol groups, such as the product Amercell Polymer HM-1500 (polyethylene glycol (15) ether of nonyl phenol) sold by the company Amerchol,
   - and mixtures thereof.
(2) hydroxypropyl guars modified with groups including at least one fatty chain, such as the product Esaflor HM 22 (C₂₂ alkyl chain) sold by the company Lamberti, and the products RE210-18 (C₁₄ alkyl chain) and RE205-1 (C₂₀ alkyl chain) sold by the company Rhodia.

The associative polysaccharides that may be used according to the invention may be chosen from cationic associative polysaccharides, notably cationic associative celluloses and cationic associative galactomannan gums.

The cationic associative celluloses may be chosen from quaternized cellulose derivatives, and in particular quaternized celluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30 carbon atoms, better still from 10 to 24, or even from 10 to 14, carbon atoms; or mixtures thereof.

Preferably, mention may be made of quaternized hydroxyethylcelluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30 carbon atoms, better still from 10 to 24, or even from 10 to 14, carbon atoms; or mixtures thereof.

Preferentially, mention may be made of the hydroxyethylcelluloses of formula (Ib): 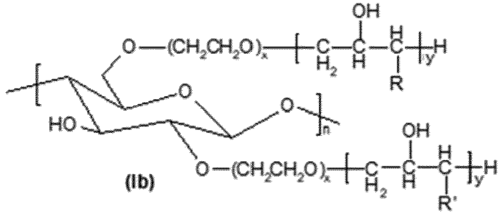 in which:
- R represents an ammonium group RaRbRcN⁺-, Q⁻ in which Ra, Rb and Rc, which may be identical or different, represent a hydrogen atom or a linear or branched C₁ to C₃₀ alkyl, preferably an alkyl, and Q⁻ represents an anionic counterion such as a halide, for instance a chloride or bromide;
- R' represents an ammonium group R'aR'bR'cN⁺-, Q'⁻ in which R'a, R'b and R'c, which may be identical or different, represent a hydrogen atom or a linear or branched C₁ to C₃₀ alkyl, preferably an alkyl, and Q'⁻ represents an anionic counterion such as a halide, for instance a chloride or bromide;
   it being understood that at least one of the radicals Ra, Rb, Rc, R'a, R'b and R'c represents a linear or branched C₈ to C₃₀ alkyl;
- n, x and y, which may be identical or different, represent an integer between 1 and 10 000.

Preferably, in formula (Ib), at least one of the radicals Rₐ, Rb, Rc, R'a, R'b or R'c represents a linear or branched C₈ to C₃₀, better still C₁₀ to C₂₄ or even C₁₀ to C₁₄ alkyl; mention may be made in particular of the dodecyl radical (C₁₂). Preferably, the other radical(s) represent a linear or branched C₁-C₄ alkyl, notably methyl.

Preferably, in formula (Ib), only one of the radicals Ra, Rb, Rc, R'a, R'b or R'c represents a linear or branched C₈ to C₃₀, better still C₁₀ to C₂₄ or even C₁₀ to C₁₄ alkyl; mention may be made in particular of the dodecyl radical (C₁₂). Preferably, the other radicals represent a linear or branched C₁ to C₄ alkyl, notably methyl.

Better still, R may be a group chosen from -N⁺(CH₃)₃, Q'⁻ and -N⁺(C₁₂H₂₅)(CH₃)₂, Q'⁻, preferably a group -N⁺(CH₃)₃, Q'⁻.

Even better still, R' may be a group -N⁺(C₁₂H₂₅)(CH₃)₂, Q'⁻.

The aryl radicals preferably denote phenyl, benzyl, naphthyl or anthryl groups.

Mention may notably be made of the polymers having the following INCI names:
- Polyquaternium-24, such as the product Quatrisoft LM 200^{®}, sold by the company Amerchol/Dow Chemical;
- PG-Hydroxyethylcellulose Cocodimonium Chloride, such as the product Crodacel QM^{®};
- PG-Hydroxyethylcellulose Lauryldimonium Chloride (C₁₂ alkyl), such as the product Crodacel QL^{®}; and
- PG-Hydroxyethylcellulose Stearyldimonium Chloride (C₁₈ alkyl), such as the product Crodacel QS^{®}, sold by the company Croda.

Mention may also be made of the hydroxyethylcelluloses of formula (Ib) in which R represents a trimethylammonium halide and R' represents a dimethyldodecylammonium halide, preferentially R represents trimethylammonium chloride (CH₃)₃N⁺-, Cl⁻ and R' represents dimethyldodecylammonium chloride (CH₃)₂(C₁₂H₂₅)N⁺-, Cl⁻. This type of polymer is known under the INCI name Polyquaternium-67; as commercial products, mention may be made of the Softcat Polymer SL^{®} polymers, such as SL-100, SL-60, SL-30 and SL-5, from the company Amerchol/Dow Chemical.

More particularly, the polymers of formula (Ib) are, for example, those whose viscosity is between 2000 and 3000 cPs inclusive, preferentially between 2700 and 2800 cPs. Typically, Softcat Polymer SL-5 has a viscosity of 2500 cPs, Softcat Polymer SL-30 has a viscosity of 2700 cPs, Softcat Polymer SL-60 has a viscosity of 2700 cPs and Softcat Polymer SL-100 has a viscosity of 2800 cPs. Use may also be made of Softcat Polymer SX-1300X with a viscosity of between 1000 and 2000 cPs.

According to a preferred embodiment of the invention, the associative polysaccharide(s) are chosen from cationic associative celluloses; more preferentially from quaternized celluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30, better still from 10 to 24, or even from 10 to 14 carbon atoms, or mixtures thereof.

Most particularly preferably, in this embodiment, the associative polysaccharide is Polyquaternium-67.

According to this embodiment, the total content of associative polysaccharide(s) present in the composition according to the invention is preferably between 0.01% and 10% by weight, more preferentially between 0.05% and 5% by weight and even more preferentially between 0.1% and 1% by weight, relative to the total weight of the composition.

Preferably, the polysaccharide(s) (iv) are chosen from microbial gums, cationic associative celluloses, and mixtures thereof; more preferentially from microbial gums, quaternized celluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30, better still from 10 to 24, or even from 10 to 14 carbon atoms, and mixtures thereof; even more preferentially from xanthan gum, scleroglucan gum, Polyquaternium-67, and mixtures thereof.

Preferably, the total content of polysaccharide(s) (iv) present in the composition according to the invention is between 0.01% and 10% by weight, more preferentially between 0.05% and 5% by weight, even more preferentially between 0.1% and 1% by weight, relative to the total weight of the composition.

### The non-oxyalkylenated and non-glycerolated fatty alcohols

Preferably, the composition according to the invention also comprises one or more non-oxyalkylenated and non-glycerolated C₈ to C₃₀ fatty alcohols.

The non-oxyalkylenated and non-glycerolated fatty alcohols comprise from 8 to 30 carbon atoms. They may be linear or branched, and also saturated or unsaturated.

The saturated fatty alcohols that may be used according to the invention may be linear or branched. They may optionally comprise in their structure at least one aromatic or non-aromatic ring. Preferably, they are acyclic. More particularly, said saturated fatty alcohols are chosen from octyldodecanol, isostearyl alcohol, 2-hexyldecanol, and also palmityl, myristyl, cetyl, stearyl and lauryl alcohols, and mixtures thereof.

The unsaturated fatty alcohols that may be used according to the invention contain in their structure at least one double or triple bond, and preferably one or more double bonds. When several double bonds are present, there are preferably 2 or 3 of them, and they may be conjugated or unconjugated. They may optionally comprise in their structure at least one aromatic or non-aromatic ring. Preferably, they are acyclic. More particularly, the unsaturated fatty alcohols are chosen from oleyl alcohol, linoleyl alcohol, linolenyl alcohol, undecylenyl alcohol, cetyl alcohol and stearyl alcohol, and mixtures thereof.

According to a preferred embodiment of the invention, the composition also comprises one or more non-oxyalkylenated and non-glycerolated C₈ to C₃₀ fatty alcohols, more preferentially chosen from oleyl alcohol, linoleyl alcohol, linolenyl alcohol, cetyl alcohol, stearyl alcohol, and mixtures thereof.

Preferably, when one or more non-oxyalkylenated and non-glycerolated C₈ to C₃₀ fatty alcohols are present in the composition according to the invention, the total content of non-oxyalkylenated and non-glycerolated C₈ to C₃₀ fatty alcohol(s) is between 0.1% and 15% by weight, more preferentially between 0.5% and 10% by weight, and even more preferentially between 1% and 5% by weight, relative to the total weight of the composition.

### The structuring agents

Preferably, the composition according to the present invention also comprises one or more structuring agents, i.e. one or more agents capable of thickening or even gelling the final composition, in particular by thickening the oils.

According to a preferred embodiment of the invention, the composition also comprises one or more structuring agents chosen from dextrin palmitate, dextrin myristate, sorbitol/sebacic acid/behenate copolymer, and mixtures thereof.

Preferably, when one or more structuring agents are present in the composition according to the invention, the content of structuring agent(s) is between 0.1% and 5% by weight.

### The polyols

Preferably, the composition according to the present invention also comprises one or more C₁ to C₇ polyols.

For the purposes of the present invention, the term "C₁ to C₇ polyol" means an organic compound consisting of a hydrocarbon-based chain comprising from 1 to 7 carbon atoms, optionally interrupted with one or more oxygen atoms and bearing at least two free hydroxyl (-OH) groups borne by different carbon atoms, this compound possibly being cyclic or acyclic, linear or branched, saturated or unsaturated, and in liquid form at room temperature (25°C) and at atmospheric pressure.

Preferably, the polyol(s) according to the invention are acyclic and non-aromatic.

The polyols according to the invention comprise in their structure a number of carbon atoms preferably less than 6, more preferentially ranging from 2 to 5, even more preferentially ranging from 2 to 4 carbon atoms.

The C₁ to C₇ polyols according to the invention are different from the non-oxyalkylenated and non-glycerolated C₈ to C₃₀ fatty alcohols described previously.

More particularly, the C₁ to C₇ polyol(s) that may be used according to the invention comprise from 2 to 10 hydroxyl groups, more preferentially from 2 to 5 hydroxyl groups and even more preferentially from 2 to 3 hydroxyl groups.

According to a preferred embodiment of the invention, the polyol(s) that may be used according to the invention are chosen from polyols comprising at least three carbon atoms, ethylene glycol, and mixtures thereof; more preferentially from propylene glycol, 1,3-propanediol, 1,3-butylene glycol, 1,2-pentanediol, dipropylene glycol, hexylene glycol, pentylene glycol, glycerol, ethylene glycol, and mixtures thereof.

Preferably, when one or more polyols are present in the composition according to the invention, the content of C₁ to C₇ polyol(s) is between 0.1% and 15% by weight, more preferentially between 1% and 15% by weight, even more preferentially between 2% and 10% by weight, relative to the total weight of the composition.

### C₁ to C₆ monoalcohols

The composition according to the invention comprises one or more C₁ to C₆ monoalcohols.

For the purposes of the invention, the term "*C₁ to* C₆ *monoalcohol"* means an aliphatic compound, preferably an alkane, comprising from 1 to 6 carbon atoms and only one hydroxyl radical -OH.

The C₁ to C₆ monoalcohols according to the invention are different from the C₁ to C₇ polyols and from the non-oxyalkylenated and non-glycerolated C₈ to C₃₀ fatty alcohols described previously.

Preferably, the C₁ to C₆ monoalcohol(s) are chosen from ethanol, isopropanol, *n*-propanol, *n*-butanol, isobutanol, *tert*-butanol, and mixtures thereof.

More preferentially, the composition according to the invention comprises ethanol.

Preferably, the total content of C₁ to C₆ monoalcohol(s) is between 10% and 50% by weight, more preferentially between 15% and 40% by weight and even more preferentially between 20% and 35% by weight, relative to the total weight of the composition according to the invention.

The composition according to the invention comprises water.

Preferably, the water content is between 30% and 70% by weight, more preferentially between 30% and 60% by weight, even more preferentially between 30% and 50% by weight, relative to the total weight of the composition.

Preferably, the pH of the composition is between 3 and 7, more preferentially between 3.5 and 7 and even more preferentially between 4 and 6.5.

The pH of these compositions may be adjusted to the desired value by means of basifying agents or acidifying agents that are usually used. Among the basifying agents, examples that may be mentioned include aqueous ammonia, alkanolamines, and mineral or organic hydroxides. Among the acidifying agents, examples that may be mentioned include mineral or organic acids, for instance hydrochloric acid or orthophosphoric acid, carboxylic acids, for instance acetic acid, tartaric acid, citric acid or lactic acid, and sulfonic acids.

The composition according to the invention may also contain additives used in cosmetics, such as preserving agents, fragrances, colorants and pigments.

These additives may be present in the composition according to the invention in an amount ranging from 0 to 20% by weight, relative to the total weight of the composition.

A person skilled in the art will take care to select these optional additives and amounts thereof so that they do not damage the properties of the compositions of the present invention.

Preferably, the composition according to the invention is in the form of a milk, a lotion or a gel. More preferentially, the composition according to the invention is in the form of a milk.

Another subject of the invention also relates to the use of the composition as defined previously, for the cosmetic treatment of, more preferentially for caring for and/or conditioning, keratin fibres, in particular human keratin fibres such as the hair.

The composition may be used on wet or dry hair, in rinse-out or leave-in mode.

Preferably, the keratin fibres are not rinsed after the application of the composition according to the invention.

A subject of the invention is also a cosmetic process for treating, preferably for caring for and/or conditioning, keratin fibres, in particular human keratin fibres such as the hair, comprising at least one step of applying to said keratin fibres a composition as described previously.

The composition may be applied to wet or dry hair, in rinse-out or leave-in mode.

Preferably, the keratin fibres are not rinsed after the step(s) of applying a composition according to the invention to said fibres.

The examples that follow serve to illustrate the invention without, however, being limiting in nature.

### Examples

### Example 1:

The cosmetic compositions (A) and (B) according to the invention are prepared from the ingredients shown in the tables below, the amounts of which are expressed as weight percentages of active material (AM).

| | Composition A |
|---|---|
| Jojoba oil (*Simmondsia chinensis seed oil*) | 8.5 |
| Dodecane | 12.5 |
| Polyquaternium-67 | 0.2 |
| Polyglyceryl-3 diisostearate | 3 |
| Ethanol | 30 |
| Water | qs 100 |

| | Composition B |
|---|---|
| Jojoba oil (*Simmondsia chinensis seed oil*) | 8.5 |
| Dodecane | 12.5 |
| Xanthan gum | 0.075 |
| Scleroglucan gum | 0.225 |
| Polyglyceryl-3 diisostearate | 3 |
| Oleyl alcohol | 1 |
| Propylene glycol | 5 |
| Ethanol | 25 |
| Water | qs 100 |

Compositions (A) and (B) according to the invention were each applied to locks of sensitized hair of Caucasian type (SA20), at a rate of 0.075 g of composition per gram of hair.

A sensory evaluation of the disentangling of the hair, of the cleanliness of the hair, of the smooth feel and smooth look, and of the sheen is then performed by three experts on each of the non-rinsed locks.

At T₀, it is observed that the locks of hair treated with one of the compositions (A) or (B) according to the invention have a good level of care/conditioning, notably in terms of the disentangling, sheen, cleanliness, smooth feel and smooth look of the hair.

### Example 2:

The cosmetic compositions (C) and (D) are prepared from the ingredients shown in the tables below, the amounts of which are expressed as weight percentages of active material (AM).

| | Composition C (Comparative) | Composition D (Invention) |
|---|---|---|
| Jojoba oil (*Simmondsia chinensis seed oil*) | 2.5 | 3.5 |
| Dodecane | 1.5 | 2.5 |
| Polyquaternium-67 | 0.2 | 0.2 |
| Polyglyceryl-3 diisostearate | 3 | 3 |
| Ethanol | 30 | 30 |
| Water | qs 100 | qs 100 |
| *Sum of the total content of plant oil(s) and of the total content of hydrocarbon-based oil(s)* | **4** | **6** |

Compositions (C) and (D) were each applied to locks of sensitized hair of Caucasian type (SA20), at a rate of 0.042 g of composition per lock of hair of 2.7 g.

The compositions were distributed homogeneously from the roots to the ends of the hair.

The locks of hair were then dried for 15 minutes in the open air.

A sensory evaluation of the smooth feel and of the lightness is then performed on dry hair by six experts, in a blind test, on each of the non-rinsed locks.

The experts evaluated using a rating scale ranging from 0 (very poor) to 5 (very good). The rating scale varies in steps of 0.5.

To evaluate the smooth feel, the expert grasps the lock between the thumb and forefinger, and slides his fingers along the lock from the roots to the ends. He evaluates whether the hair is soft, if it does not have any roughness, if it does not hold the fingers, if the touch is homogeneous.

To evaluate the lightness, the expert lifts the hair in large strands with the hands and looks at the way the hair falls: the light hair is individualized, flowing and does not fall out in clumps.

The results are summarized in the tables below:

| *LIGHTNESS* | Composition C (Comparative) | Composition D (Invention) |
|---|---|---|
| Expert 1 | 2 | 4 |
| Expert 2 | 2 | 4 |
| Expert 3 | 2 | 4 |
| Expert 4 | 3 | 4 |
| Expert 5 | 2 | 4 |
| Expert 6 | 3.5 | 3 |
| **Average** | **2.4** | **3.8** |
| Standard deviations | 0.7 | 0.4 |

| *SMOOTH FEEL* | Composition C (Comparative) | Composition D (Invention) |
|---|---|---|
| Expert 1 | 3 | 3.5 |
| Expert 2 | 3 | 3.5 |
| Expert 3 | 3 | 4 |
| Expert 4 | 3 | 4 |
| Expert 5 | 3 | 4 |
| Expert 6 | 3.5 | 4 |
| **Average** | **3.1** | **3.8** |
| Standard deviations | 0.2 | 0.3 |

It is observed that the locks of hair treated with the composition (D) according to the invention are lighter and smoother than the locks of hair treated with the comparative composition (C).

## Claims

1. Composition comprising:
(i) one or more plant oils,
(ii) one or more hydrocarbon-based oils other than the plant oils (i),
(iii) one or more glycerolated nonionic surfactants,
(iv) one or more polysaccharides,
(v) one or more C₁ to C₆ monoalcohols and
(vi) water; and
the sum of the total content of plant oil(s) (i) and of the total content of hydrocarbon-based oil(s) (ii) being greater than or equal to 5% by weight relative to the total weight of the composition.

2. Composition according to the preceding claim, **characterized in that** the plant oil(s) (i) are chosen from sweet almond oil, argan oil, avocado oil, groundnut oil, camela oil, safflower oil, beauty-leaf oil, rapeseed oil, coconut oil, coriander oil, marrow oil, wheat germ oil, jojoba oil, linseed oil, macadamia oil, corn germ oil, hazelnut oil, walnut oil, vernonia oil, apricot kernel oil, olive oil, evening-primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil, and mixtures thereof; preferably from soybean oil, jojoba oil, castor oil, coconut oil, and mixtures thereof.

3. Composition according to either of the preceding claims, **characterized in that** the hydrocarbon-based oil(s) (ii) have a surface tension, at 25°C and at 1.013 × 10⁵ Pa, of less than or equal to 50 mN/m, preferably less than or equal to 30 mN/m.

4. Composition according to any one of the preceding claims, **characterized in that** the hydrocarbon-based oil(s) (ii) are chosen from C₈ to C₁₆ hydrocarbon-based oils; preferably from branched C₈ to C₁₆ alkanes such as isododecane, isodecane and isohexadecane, linear C₈ to C₁₆ alkanes such as n-dodecane and n-tetradecane, and mixtures thereof.

5. Composition according to any one of the preceding claims, **characterized in that** the sum of the total content of plant oil(s) (i) and of the total content of hydrocarbon-based oil(s) (ii) is between 10% and 40% by weight, more preferentially between 15% and 35% by weight and even more preferentially between 15% and 25% by weight, relative to the total weight of the composition.

6. Composition according to any one of the preceding claims, **characterized in that** the glycerolated nonionic surfactant(s) are chosen from:
- monoesters or polyesters of linear or branched, monoglycerolated or polyglycerolated C₈ to C₄₀ acids, comprising from 1 to 50 mol of glycerol, preferably from 1 to 20 or even from 2 to 10 mol of glycerol; in particular monoesters or diesters of linear or branched C₈ to C₃₂, better still C₁₀ to C₂₈ or even C₁₀ to C₂₄ acids, comprising from 1 to 50 mol of glycerol, preferably from 1 to 20 or even from 2 to 10 mol of glycerol,
- linear or branched, saturated or unsaturated, monoglycerolated or polyglycerolated C₈ to C₄₀, better still C₁₀ to C₂₈, even better still C₁₀ to C₂₄ or even C₁₀ to C₁₈ alcohols, preferably including one or two fatty chains, and comprising from 1 to 50 mol of glycerol, preferably from 1 to 20 or even from 2 to 10 mol of glycerol,
- mixtures thereof;
and more preferentially from monoesters or diesters of linear or branched, monoglycerolated or polyglycerolated C₈ to C₄₀, better still C₈ to C₃₂, even better still C₁₀ to C₂₈ or even C₁₀ to C₂₄ acids, comprising from 1 to 50 mol of glycerol, preferably from 1 to 20 or even from 2 to 10 mol of glycerol, and mixtures thereof.

7. Composition according to any one of the preceding claims, **characterized in that** the polysaccharide(s) are chosen from microbial gums, cationic associative celluloses, and mixtures thereof; preferably from microbial gums, quaternized celluloses modified with groups including at least one fatty chain, such as linear or branched alkyl groups, linear or branched arylalkyl groups, or linear or branched alkylaryl groups, preferably linear or branched alkyl groups, these groups including at least 8 carbon atoms, notably from 8 to 30, better still from 10 to 24, or even from 10 to 14 carbon atoms, and mixtures thereof; more preferentially from xanthan gum, scleroglucan gum, Polyquaternium-67, and mixtures thereof.

8. Composition according to any one of the preceding claims, **characterized in that** the total content of C₁ to C₆ monoalcohol(s) is between 10% and 50% by weight, preferably between 15% and 40% by weight, and more preferentially between 20% and 35% by weight, relative to the total weight of the composition.

9. Composition according to any one of the preceding claims, **characterized in that** the weight ratio of the total content of plant oil(s) (i), on the one hand, to the total content of glycerolated nonionic surfactant(s) (iii), on the other hand, is greater than or equal to 1, preferably greater than or equal to 2 and more preferentially between 2 and 4.

10. Composition according to any one of the preceding claims, **characterized in that** it also comprises one or more non-oxyalkylenated and non-glycerolated C₈ to C₃₀ fatty alcohols, preferentially chosen from oleyl alcohol, linoleyl alcohol, linolenyl alcohol, cetyl alcohol, stearyl alcohol, and mixtures thereof.

11. Composition according to any one of the preceding claims, **characterized in that** it also comprises one or more structuring agents, preferably chosen from dextrin palmitate, dextrin myristate, sorbitol/sebacic acid/behenate copolymer, and mixtures thereof.

12. Composition according to any one of the preceding claims, **characterized in that** it also comprises one or more C₁ to C₇ polyols, preferably chosen from polyols comprising at least three carbon atoms, ethylene glycol, and mixtures thereof; more preferentially from propylene glycol, 1,3-propanediol, 1,3-butylene glycol, 1,2-pentanediol, dipropylene glycol, hexylene glycol, pentylene glycol, glycerol, ethylene glycol, and mixtures thereof.

13. Composition according to any one of the preceding claims, wherein the total content of silicone(s) is less than or equal to 0.1% by weight, preferably is less than or equal to 0.05% by weight, and more preferentially is equal to 0% by weight, relative to the total weight of the composition.

14. Cosmetic process for treating keratin fibres, preferably for caring for and/or conditioning keratin fibres, comprising at least one step of applying to said keratin fibres the composition as defined in any one of Claims 1 to 13.

15. Use of the composition as defined in any one of Claims 1 to 13, for the cosmetic treatment of keratin fibres, preferably for caring for and/or conditioning keratin fibres.

## Patentansprüche

1. Zusammensetzung, umfassend:
(i) ein oder mehrere Pflanzenöle,
(ii) ein oder mehrere Öle auf Kohlenwasserstoffbasis, die von den Pflanzenölen (i) verschieden sind,
(iii) ein oder mehrere glycerinierte nichtionische Tenside,
(iv) ein oder mehrere Polysaccharide,
(v) einen oder mehrere C₁- bis C₆-Monoalkohole und
(vi) Wasser; und
wobei die Summe des Gesamtgehalts an Pflanzenöl(en) (i) und des Gesamtgehalts an Öl(en) auf Kohlenwasserstoffbasis (ii) größer oder gleich 5 Gew.-%, bezogen auf das Gesamtgewicht der Zusammensetzung, ist.

2. Zusammensetzung nach dem vorhergehenden Anspruch, **dadurch gekennzeichnet, dass** das Pflanzenöl bzw. die Pflanzenöle (i) aus Süßmandelöl, Arganöl, Avocadoöl, Erdnussöl, Kamelienöl, Distelöl, Schönblattöl, Rapsöl, Kokosnussöl, Korianderöl, Kürbisöl, Weizenkeimöl, Jojobaöl, Leinsamenöl, Macadamiaöl, Maiskeimöl, Haselnussöl, Walnussöl, Vernoniaöl, Aprikosenkernöl, Olivenöl, Nachtkerzenöl, Palmöl, Passionsblumenöl, Traubenkernöl, Rosenöl, Rizinusöl, Roggenöl, Sesamöl, Reiskleieöl, Leindotteröl, Sojabohnenöl, Sonnenblumenöl, Pracaxiöl, Babassuöl, Mongongoöl, Marulaöl, Araraöl, Sheabutteröl, Paranussöl und Mischungen davon und vorzugsweise aus Sojabohnenöl, Jojobaöl, Rizinusöl, Kokosnussöl und Mischungen davon ausgewählt ist bzw. sind.

3. Zusammensetzung nach einem der vorhergehenden Ansprüche, **dadurch gekennzeichnet, dass** das Öl bzw. die Öle auf Kohlenwasserstoffbasis (ii) eine Oberflächenspannung bei 25 °C und bei 1,013x10⁵ Pa kleiner oder gleich 50 mN/m, vorzugsweise kleiner oder gleich 30 mN/m, aufweist bzw. aufweisen.

4. Zusammensetzung nach einem der vorhergehenden Ansprüche, **dadurch gekennzeichnet, dass** das Öl bzw. die Öle auf Kohlenwasserstoffbasis (ii) aus Ölen auf C₈- bis C₁₆-Kohlenwasserstoffbasis und bevorzugt aus verzweigten C₈- bis C₁₆-Alkanen wie Isododecan, Isodecan und Isohexadecan, linearen C₈- bis C₁₆-Alkanen wie n-Dodecan und n-Tetradecan sowie Mischungen davon ausgewählt ist bzw. sind.

5. Zusammensetzung nach einem der vorhergehenden Ansprüche, **dadurch gekennzeichnet, dass** die Summe des Gesamtgehalts an Pflanzenöl(en) (i) und des Gesamtgehalts an Öl(en) auf Kohlenwasserstoffbasis (ii) zwischen 10 und 40 Gew.-%, weiter bevorzugt zwischen 15 und 35 Gew.-% und noch weiter bevorzugt zwischen 15 und 25 Gew.-%, bezogen auf das Gesamtgewicht der Zusammensetzung, liegt.

6. Zusammensetzung nach einem der vorhergehenden Ansprüche, **dadurch gekennzeichnet, dass** das glycerinierte nichtionische Tensid bzw. die glycerinierten nichtionischen Tenside aus Folgendem ausgewählt ist bzw. sind:
- Monoestern oder Polyestern von linearen oder verzweigten, monoglycerinierten oder polyglycerinierten C₈- bis C₄₀-Säuren, die 1 bis 50 mol Glycerin, vorzugsweise 1 bis 20 oder sogar 2 bis 10 mol Glycerin umfassen; insbesondere Monoestern oder Diestern von linearen oder verzweigten C₈- bis C₃₂-, noch besser C₁₀-bis C₂₈- oder sogar C₁₀- bis C₂₄-Säuren, die 1 bis 50 mol Glycerin, vorzugsweise 1 bis 20 oder sogar 2 bis 10 mol Glycerin umfassen,
- linearen oder verzweigten, gesättigten oder ungesättigten, monoglycerinierten oder polyglycerinierten C₈- bis C₄₀-, noch besser C₁₀- bis C₂₈-, sogar noch besser C₁₀- bis C₂₄- oder sogar C₁₀- bis C₁₈-Alkoholen, die vorzugsweise eine oder zwei Fettketten enthalten und 1 bis 50 mol Glycerin, vorzugsweise 1 bis 20 oder sogar 2 bis 10 mol Glycerin umfassen,
- Mischungen davon;
und weiter bevorzugt aus Monoestern oder Diestern von linearen oder verzweigten, monoglycerinierten oder polyglycerinierten C₈- bis C₄₀-, noch besser C₈- bis C₃₂-, sogar noch besser C₁₀- bis C₂₈- oder sogar C₁₀- bis C₂₄-Säuren, die 1 bis 50 mol Glycerin, vorzugsweise 1 bis 20 oder sogar 2 bis 10 mol Glycerin umfassen, und Mischungen davon.

7. Zusammensetzung nach einem der vorhergehenden Ansprüche, **dadurch gekennzeichnet, dass** das Polysaccharid bzw. die Polysaccharide aus mikrobiellen Gummen, kationischen assoziativen Cellulosen und Mischungen davon, vorzugsweise aus mikrobiellen Gummen, quaternisierten Cellulosen, die mit Gruppen mit mindestens einer Fettkette, wie linearen oder verzweigten Alkylgruppen, linearen oder verzweigten Arylalkylgruppen oder linearen oder verzweigten Alkylarylgruppen, vorzugsweise linearen oder verzweigten Alkylgruppen, wobei diese Gruppen mindestens 8 Kohlenstoffatome, insbesondere 8 bis 30, noch besser 10 bis 24 oder sogar 10 bis 14 Kohlenstoffatome enthalten, modifiziert sind, und Mischungen davon und weiter bevorzugt aus Xanthangummi, Skleroglucan-Gummen, Polyquaternium-67 und Mischungen davon, ausgewählt ist bzw. sind.

8. Zusammensetzung nach einem der vorhergehenden Ansprüche, **dadurch gekennzeichnet, dass** der Gesamtgehalt an C₁- bis C₆-Monoalkohol(en) zwischen 10 und 50 Gew.-%, vorzugsweise zwischen 15 und 40 Gew.-% und weiter bevorzugt zwischen 20 und 35 Gew.-%, bezogen auf das Gesamtgewicht der Zusammensetzung, liegt.

9. Zusammensetzung nach einem der vorhergehenden Ansprüche, **dadurch gekennzeichnet, dass** das Gewichtsverhältnis des Gesamtgehalts an Pflanzenöl (en) (i) einerseits zum Gesamtgehalt an glyceriniertem/n nichtionischem/n Tensid(en) (iii) andererseits größer oder gleich 1 und vorzugsweise größer oder gleich 2 ist und weiter bevorzugt zwischen 2 und 4 liegt.

10. Zusammensetzung nach einem der vorhergehenden Ansprüche, **dadurch gekennzeichnet, dass** sie außerdem einen oder mehrere nicht oxyalkylierte und nicht glycerinierte C₈- bis C₃₀-Fettalkohole umfasst, die vorzugsweise aus Oleylalkohol, Linoleylalkohol, Linolenylalkohol, Cetylalkohol, Stearylalkohol und Mischungen davon ausgewählt sind.

11. Zusammensetzung nach einem der vorhergehenden Ansprüche, **dadurch gekennzeichnet, dass** sie außerdem ein oder mehrere Strukturierungsmittel enthält, die vorzugsweise aus Dextrinpalmitat, Dextrinmyristat, Sorbit/Sebacinsäure/Behenat-Copolymer und Mischungen davon ausgewählt sind.

12. Zusammensetzung nach einem der vorhergehenden Ansprüche, **dadurch gekennzeichnet, dass** sie außerdem ein oder mehrere C₁- bis C₇-Polyole umfasst, die vorzugsweise aus Polyolen mit mindestens drei Kohlenstoffatomen, Ethylenglykol und Mischungen davon und weiter bevorzugt aus Propylenglykol, 1,3-Propandiol, 1,3-Butylenglykol, 1,2-Pentandiol, Dipropylenglykol, Hexylenglykol, Pentylenglykol, Glycerin, Ethylenglykol und Mischungen davon ausgewählt sind.

13. Zusammensetzung nach einem der vorhergehenden Ansprüche, wobei der Gesamtgehalt an Silikon(en) kleiner oder gleich 0,1 Gew.-%, vorzugsweise kleiner oder gleich 0,05 Gew.-% und weiter bevorzugt gleich 0 Gew.-% ist, bezogen auf das Gesamtgewicht der Zusammensetzung.

14. Kosmetisches Verfahren zur Behandlung von Keratinfasern, vorzugsweise zum Pflegen und/oder Konditionieren von Keratinfasern, umfassend mindestens einen Schritt des Aufbringens der Zusammensetzung gemäß einem der Ansprüche 1 bis 13 auf die Keratinfasern.

15. Verwendung der Zusammensetzung gemäß einem der Ansprüche 1 bis 13 zur kosmetischen Behandlung von Keratinfasern, vorzugsweise zum Pflegen und/oder Konditionieren von Keratinfasern.

## Revendications

1. Composition comprenant :
(i) une ou plusieurs huiles végétales,
(ii) une ou plusieurs huiles hydrocarbonées différentes des huiles végétales (i),
(iii) un ou plusieurs tensioactifs non ioniques glycérolés,
(iv) un ou plusieurs polysaccharides,
(v) un ou plusieurs monoalcools en C₁ à C₆, et
(vi) de l'eau ; et
la somme de la teneur totale en huile(s) végétale(s) (i) et de la teneur totale en huile(s) hydrocarbonée(s) (ii) étant supérieure ou égale à 5% en poids, par rapport au poids total de la composition.

2. Composition selon la revendication précédente, **caractérisée en ce que** la ou les huiles végétales (i) sont choisies parmi l'huile d'amande douce, l'huile d'argan, l'huile d'avocat, l'huile d'arachide, l'huile de camélia, l'huile de carthame, l'huile de calophyllum, l'huile de colza, l'huile de coco, l'huile de coriandre, l'huile de courge, l'huile de germes de blé, l'huile de jojoba, l'huile de lin, l'huile de macadamia, l'huile de germes de maïs, l'huile de noisette, l'huile de noix, l'huile de vernonia, l'huile de noyau d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de cameline, l'huile de soja, l'huile de tournesol, l'huile de pracaxi, l'huile de babassu, l'huile de mongongo, l'huile de marula, l'huile d'arara, l'huile de beurre de karité, l'huile de noix du brésil, et leurs mélanges ; de préférence parmi l'huile de soja, l'huile de jojoba, l'huile de ricin et l'huile de coco, et leurs mélanges.

3. Composition selon l'une quelconque des revendications précédentes, **caractérisée en ce que** la ou les huiles hydrocarbonées (ii) présentent une tension de surface, à 25°C et à 1,013.10⁵ Pa, inférieure ou égale à 50 mN/m ; de préférence inférieure ou égale à 30 mN/m.

4. Composition selon l'une quelconque des revendications précédentes, **caractérisée en ce que** la ou les huiles hydrocarbonées (ii) sont choisies parmi les huiles hydrocarbonées en C₈ à C₁₆ ; de préférence parmi les alcanes ramifiés en C₈ à C₁₆ tels que l'isododécane, l'isodécane et l'isohexadécane, les alcanes linéaires en C₈ à C₁₆ tels que le n-dodécane et le n-tétradécane, et leurs mélanges.

5. Composition selon l'une quelconque des revendications précédentes, **caractérisée en ce que** la somme de la teneur totale en huile(s) végétale(s) (i) et de la teneur totale en huile(s) hydrocarbonée(s) (ii) est comprise entre 10% et 40% en poids ; plus préférentiellement entre 15% et 35% en poids ; plus préférentiellement encore entre 15% et 25% en poids, par rapport au poids total de la composition.

6. Composition selon l'une quelconque des revendications précédentes, **caractérisée en ce que** le ou les tensioactifs non ioniques glycérolés sont choisis parmi :
- les mono ou polyesters d'acides en C₈ à C₄₀, linéaires ou ramifiés, mono- ou polyglycérolés, comprenant de 1 à 50 moles de glycérol, de préférence de 1 à 20, voire de 2 à 10 moles de glycérol ; en particulier les mono- ou di-esters d'acides en C₈ à C₃₂, mieux en C₁₀ à C₂₈, voire en C₁₀ à C₂₄, linéaires ou ramifiés, comprenant de 1 à 50 moles de glycérol, de préférence de 1 à 20, voire de 2 à 10 moles de glycérol,
- les alcools en C₈ à C₄₀, mieux en C₁₀ à C₂₈, encore mieux en C₁₀ à C₂₄, voire en C₁₀ à C₁₈, mono- ou polyglycérolés, saturés ou non, linéaires ou ramifiés, comportant de préférence une ou deux chaînes grasses, et comprenant de 1 à 50 moles de glycérol, de préférence de 1 à 20, voire de 2 à 10 moles de glycérol,
- leurs mélanges ;
et plus préférentiellement parmi les mono- ou di-esters d'acides en C₈ à C₄₀, mieux en C₈ à C₃₂, encore mieux en C₁₀ à C₂₈, voire en C₁₀ à C₂₄, linéaires ou ramifiés, mono- ou polyglycérolés, comprenant de 1 à 50 moles de glycérol, de préférence de 1 à 20, voire de 2 à 10 moles de glycérol, et leurs mélanges.

7. Composition selon l'une quelconque des revendications précédentes, **caractérisée en ce que** le ou les polysaccharides sont choisis parmi les gommes microbiennes, les celluloses associatives cationiques, et leurs mélanges ; de préférence parmi les gommes microbiennes, les celluloses quaternisées modifiées par des groupements comportant au moins une chaîne grasse, tels que les groupes alkyle linéaire ou ramifié, arylalkyle linéaire ou ramifié, alkylaryle linéaire ou ramifié, de préférence alkyl linéaire ou ramifié, ces groupes comportant au moins 8 atomes de carbone, notamment de 8 à 30, mieux de 10 à 24, voire de 10 à 14, atomes de carbone, et leurs mélanges ; plus préférentiellement parmi la gomme de xanthane, la gomme de scléroglucane, le polyquaternium-67, et leurs mélanges.

8. Composition selon l'une quelconque des revendications précédentes, **caractérisée en ce que** la teneur totale en monoalcool(s) en C₁ à C₆ est comprise entre 10% et 50% en poids ; de préférence entre 15% et 40% en poids ; plus préférentiellement entre 20% et 35% en poids, par rapport au poids total de la composition.

9. Composition selon l'une quelconque des revendications précédentes, **caractérisée en ce que** le rapport pondéral de la teneur totale en huile(s) végétale(s) (i) d'une part, sur la teneur totale en tensioactif(s) non ionique(s) glycérolé(s) (iii) d'autre part, est supérieur ou égale à 1, de préférence supérieur ou égale à 2 ; plus préférentiellement compris entre 2 et 4.

10. Composition selon l'une quelconque des revendications précédentes, **caractérisée en ce qu'**elle comprend en outre un ou plusieurs alcools gras en C₈ à C₃₀ non-oxyalkylénés et non-glycérolés ; de préférence choisis parmi l'alcool oléique, l'alcool linoléique, l'alcool linolénique, l'alcool cétylique, l'alcool stéarylique, et leurs mélanges.

11. Composition selon l'une quelconque des revendications précédentes, **caractérisée en ce qu'**elle comprend en outre un ou plusieurs agents structurants ; de préférence choisi parmi le palmitate de dextrine, le myristate de dextrine, le copolymère de sorbitol/acide sebacique/béhénate, et leurs mélanges.

12. Composition selon l'une quelconque des revendications précédentes, **caractérisée en ce qu'**elle comprend en outre un ou plusieurs polyols en C₁ à C₇ ; de préférence choisi parmi les polyols comprenant au moins trois atomes de carbones, l'éthylène glycol, et leurs mélanges ; plus préférentiellement parmi le propylène glycol, le 1,3-propanediol, le 1,3-butylène glycol, le pentane-1,2-diol, le dipropylène glycol, l'hexylène glycol, le pentylène glycol, le glycérol, l'éthylène glycol, et leurs mélanges.

13. Composition selon l'une quelconque des revendications précédentes, dans laquelle la teneur totale en silicone(s) est inférieure ou égale à 0,1% en poids, de préférence est inférieure ou égale à 0,05% en poids, et plus préférentiellement est égale à 0% en poids, par rapport au poids total de la composition.

14. Procédé de traitement cosmétique des fibres kératiniques, de préférence de soin et/ou de conditionnement des fibres kératiniques, comprenant au moins une étape d'application sur lesdites fibres kératiniques de la composition telle que définie dans l'une quelconque des revendications 1 à 13.

15. Utilisation de la composition telle que définie dans l'une quelconque des revendications 1 à 13, pour le traitement cosmétique des fibres kératiniques ; de préférence pour le soin et/ou le conditionnement des fibres kératiniques.
